# EUROPEAN PATENT SPECIFICATION

(11) **EP 4 242 626 B1**
(45) Date of publication and mention of the grant of the patent: **22.04.2026**
(21) Application number: 21889208.1
(22) Date of filing: 02.11.2021
(51) Int. Cl.: G01N 5/02, G01N 29/02, G01N 29/22, G01N 33/00

(54) **MEASURING DEVICE WITH ODOR SENSOR**
MESSVORRICHTUNG MIT GERUCHSSENSOR
DISPOSITIF DE MESURE AVEC CAPTEUR OLFACTIF

(30) Priority: 04.11.2020 JP 2020184741
(43) Date of publication of application: 13.09.2023
(73) Proprietor: REVORN CO., LTD., Chuo-ku Tokyo 1040033 (JP)
(72) Inventor: MATSUOKA Hiroaki, Tokyo 150-0022 (JP)
(74) Representative: Winter, Brandl - Partnerschaft mbB
(86) International application number: PCT/JP2021/040460
(87) International publication number: WO 2022/097655

(56) References cited:
- WO-A1-2018/061092
- CN-U- 205 879 922
- JP-A- 2014 119 357
- US-A1- 2015 219 608
- US-B1- 6 422 061

## Description

### BACKGROUND

### TECHNICAL FIELD

The present invention relates to a measurement apparatus.

### RELATED ART

Odor measurement is often performed by bringing air collected in a container or the like into contact with an odor sensor, as suggested in WO 2019/117099 A1). However, measuring odor by the method disclosed in WO 2019/117099 A1 requires a great deal of time and effort.

US 2015/219608 A1 discloses an electronic device having an environmental sensor and an interface unit hole, a microphone hole and a speaker hole formed on a case or body of the electronic device. The interface unit hole, a microphone hole and a speaker hole form inflow holes or outflow holes for air supplied to the the environmental sensor.

WO 2018/061092 A1 discloses an odor measurement apparatus including an odor sensor detecting an odor. The odor measurement apparatus detects odor substances contained in air using a sensor when an odor is measured, and measures attribute information of a measurement target or the like of the odor. An introduction port disposed in a housing of the measurement apparatus, for introducing air to a sensor surface is provided with a shutter for opening/closing the introduction port.

CN 205 879 922 U discloses an odor detector including a detector housing with a gas collection hole.

Further, a measurement apparatus in accordance with the features of the preamble of claim 1 is disclosed in US 6422061 B1.

### SUMMARY

The object of the present invention is to provide a measurement apparatus capable of easily performing odor measurement.

The above object is solved by the subject matter of claim 1. Preferred embodiments are subject matter of dependent claims.

According to the present invention, it is possible to easily measure odor.

### BRIEF DESCRIPTION OF DRAWINGS

FIG. 1 is a diagram showing a front appearance of a measurement apparatus.
FIG. 2 is a diagram showing a rear appearance of the measurement apparatus.
FIG. 3 is a perspective view showing an appearance of the measurement apparatus.
FIG. 4 is a diagram showing a rear surface of a front side of a housing 1.
FIG. 5 is a diagram showing an example of a substrate accommodated in the housing 1.
FIG. 6 is a diagram showing a flow of air/gas according to a first utilization embodiment.
FIG. 7 is a diagram showing a flow of air/gas according to a second utilization embodiment.

### DETAILED DESCRIPTION

Hereinafter, embodiment of the present invention will be described with reference to the drawings. Various features described in the embodiment below can be combined with each other.

A program for realizing a software in the present embodiment may be provided as a non-transitory computer readable medium that can be read by a computer or may be provided for download from an external server or may be provided so that the program can be activated on an external computer to realize functions thereof on a client terminal (so-called cloud computing).

In the present embodiment, the "unit" may include, for instance, a combination of hardware resources implemented by a circuit in a broad sense and information processing of software that can be concretely realized by these hardware resources. Further, various information is performed in the present embodiment, and the information can be represented by, for instance, physical values of signal values representing voltage and current, high and low signal values as a set of binary bits consisting of 0 or 1, or quantum superposition (so-called qubits), and communication/calculation can be performed on a circuit in a broad sense.

Further, the circuit in a broad sense is a circuit realized by combining at least an appropriate number of a circuit, a circuitry, a processor, a memory, or the like. In other words, it is a circuit includes application specific integrated circuit (ASIC), programmable logic device (e.g., simple programmable logic device (SPLD), complex programmable logic device (CPLD), field programmable gate array (FPGA)), or the like.

### 1. Appearance of the measurement apparatus

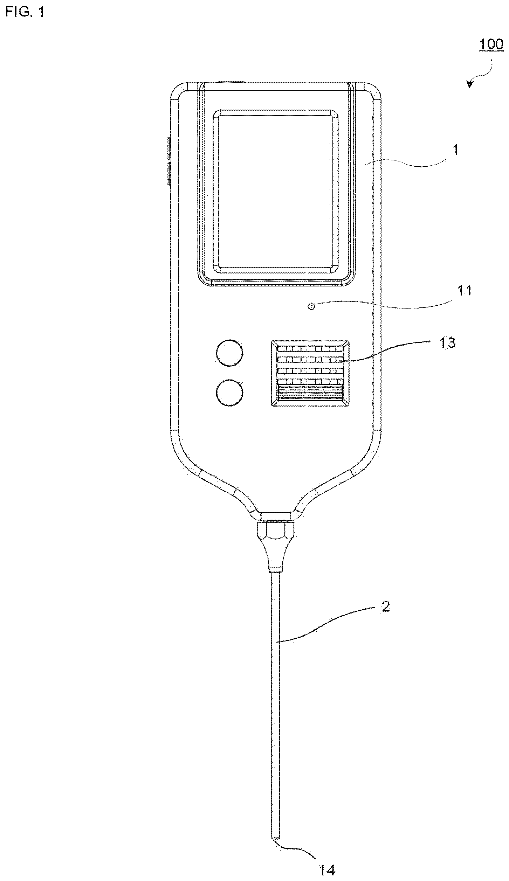

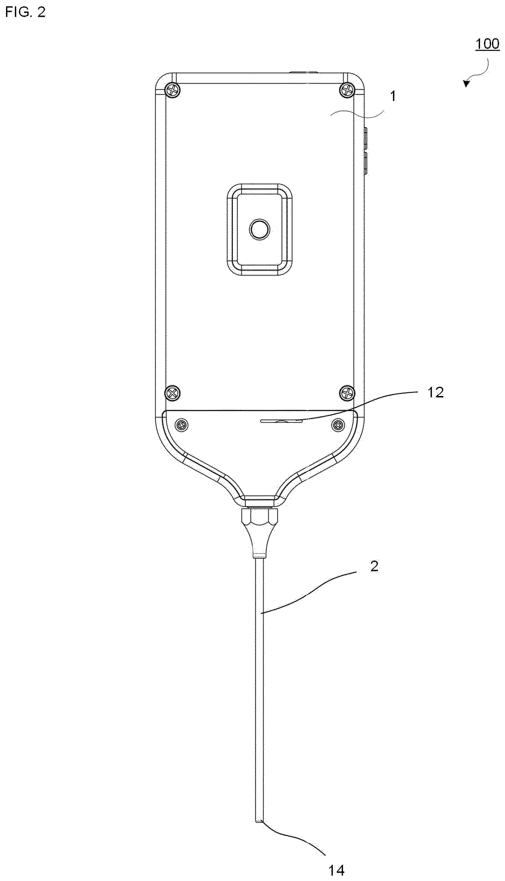

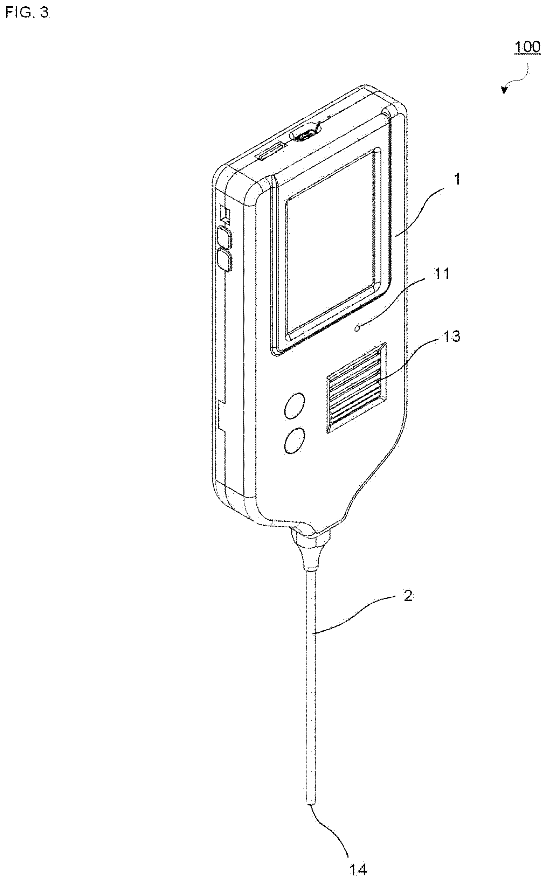

FIG. 1 is a diagram showing a front appearance of a measurement apparatus. FIG. 2 is a diagram showing a rear appearance of the measurement apparatus. Further, FIG. 3 is a perspective view showing an appearance of the measurement apparatus.

A measurement apparatus 100 shown in these drawings comprises a substrate 3 (not shown) and a housing 1. The housing 1 is connected to a nozzle 2. The housing 1 accommodates the substrate (not shown) and include at least three openings. For instance, the housing 1 includes an opening 11 that is a first opening, an opening 12 that is a second opening, an opening 13 that is a third opening, and a nozzle opening 14. Further, two or more third openings may be provided in the housing 1, and at least one of the third openings, e.g., the opening 13, may include a shutter that can be opened/closed.

The opening 11 is an air outlet. The opening 12 is an air inlet. The opening 13 and the nozzle opening 14 are inlets for gas containing a component to be measured.

### 2. Inside of housing 1

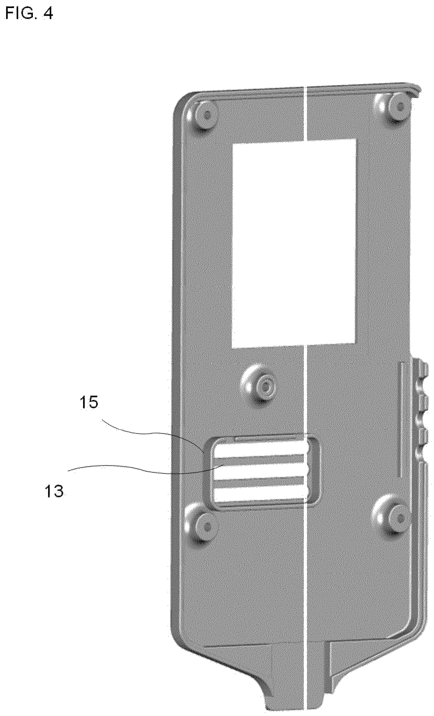

FIG. 4 is a diagram showing a rear surface of a front side of the housing 1. As shown in the drawing, an enclosure 15 may be provided on a rear surface of the opening 13. The enclosure 15 allows gas flowing from the opening 13 to flow in a vicinity of an odor sensor accommodated in the housing 1.

### 3. Substrate of measurement apparatus

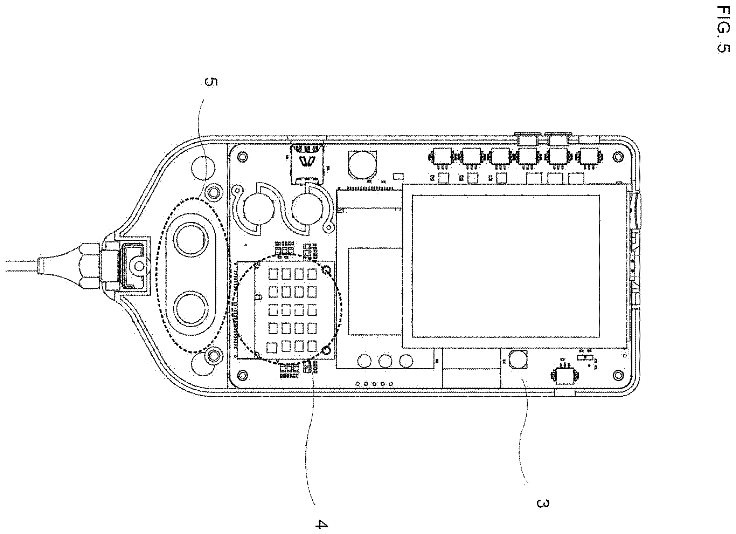

FIG. 5 is a diagram showing an example of a substrate accommodated in the housing 1. As shown in the drawing, the substrate 3 is provided with an odor sensor 4 and a pump 5. The odor sensor 4 is configured of, for instance, two or more Quartz Crystal Microbalance sensors. Further, the odor sensor 4 is arranged directly below the opening 13. The pump 5 allows gas that flows in through the opening 13 and gas that flows in through the nozzle opening 14 to be discharged from the opening 11. The odor sensor 4 may be applied with a semiconductor type such as an oxide semiconductor type or an organic semiconductor type, a quartz oscillator type using an epoxy resin film, a vinyl acetate resin film, a Langmuir-Blodgett film, or the like as a sensitive film, or other types such as using a surface acoustic wave (SAW) filter or a film bulk acoustic wave (FBAR) filter.

### 4. First utilization embodiment of measurement apparatus

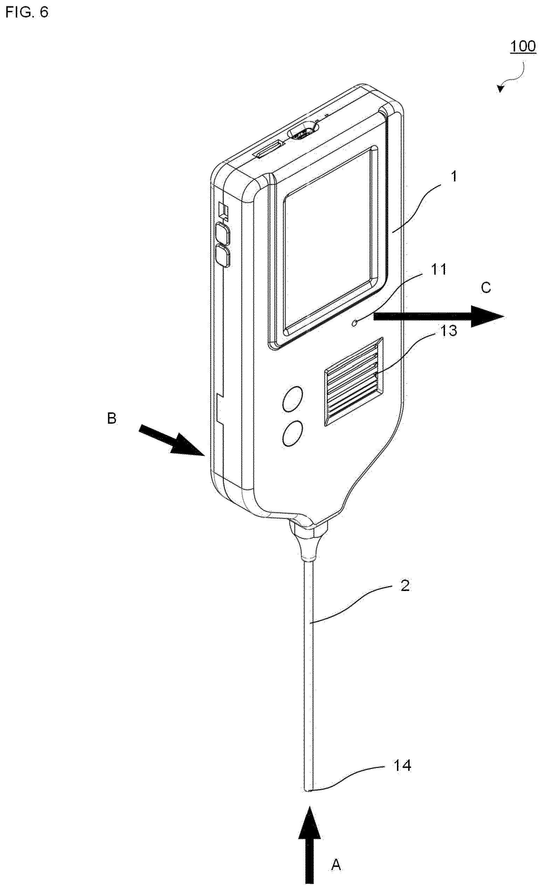

FIG. 6 is a diagram showing a flow of air/gas according to a first utilization embodiment. In the first utilization embodiment, the nozzle opening 14 is an inlet for gas containing a component to be measured, the opening 12 is an inlet for ambient air, and the opening 11 is an outlet for air. Specifically, in the utilization embodiment, the nozzle opening 14 is aimed at an object to be measured by a measurer, and air flows in from the nozzle opening 14 and flows out from the opening 11. To create such a flow of air, the substrate 3 accommodated in the housing 1 may comprise a pump 5. The pump 5 is, for instance, a piezo pump, which allows air that flows in through the opening 12 and the nozzle opening 14 to be discharged from the opening 11. In the utilization embodiment, odor in a vicinity of the measurement apparatus can be measured. At this time, the shutter of the opening 13 is closed. As a result, in the first utilization embodiment, gas that flows in as indicated by arrow A shown in the drawing and air that flows in as indicated by arrow B shown in the drawing flow out as indicated by arrow C shown in the drawing.

### 5. Second utilization embodiment of measurement apparatus

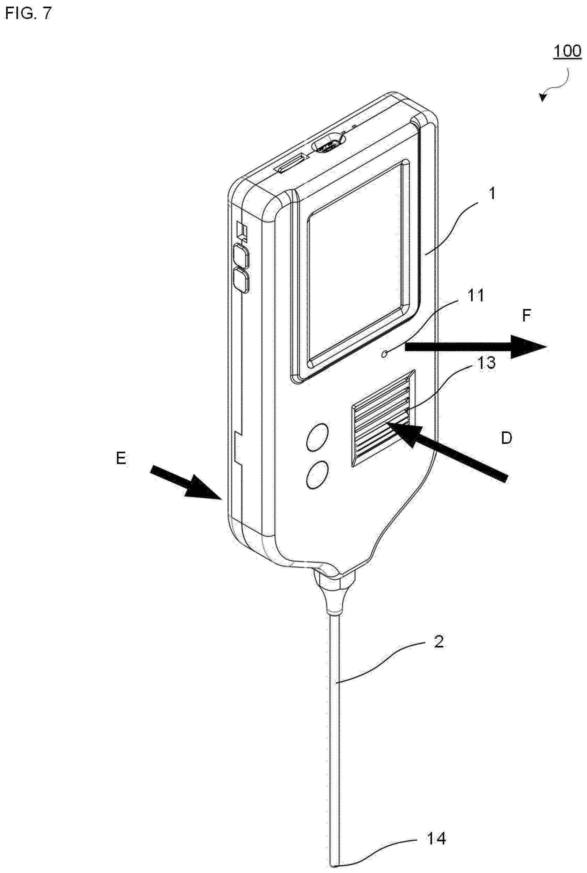

FIG. 7 is a diagram showing a flow of air/gas according to a second utilization embodiment. In the second utilization embodiment, the opening 13 is an inlet for gas containing the component to be measured, the opening 12 is an inlet for ambient air, and the opening 11 is an outlet for air. Specifically, in the utilization embodiment, atmosphere around the measurement apparatus 100 flows in through the opening 13, ambient air (for cleaning the odor sensor 4) flows in through the opening 12, and air flows out through the opening 11. To create such an air flow, the substrate 3 accommodated in the housing 1 may comprise a pump 5. The pump 5 is, for instance, a piezo pump, which allows air that flows in through the opening 12 and the opening 13 to be discharged from the opening 11. In the utilization embodiment, odor in a vicinity of the measurement apparatus 100 can be measured. As a result, in the second utilization embodiment, gas that flows in as indicated by arrow C shown in the drawing and air that flows in as indicated by arrow D shown in the drawing flow out as indicated by arrow E shown in the drawing.

### REFERENCE SIGNS LIST

1: Housing
2: Nozzle
3: Substrate
4: Sensor
5: Pump
11: Opening
12: Opening
13: Opening
14: Nozzle opening
15: Enclosure
100: Measuring apparatus
A: Arrow
B: Arrow
C: Arrow
D: Arrow
E: Arrow

## Claims

1. A measurement apparatus (100), comprising:
a substrate (3) disposed with an odor sensor (4); and
a housing (1) accommodating the substrate (3) and including a first opening (11) that is an air outlet, a second opening (12) that is an air inlet, and at least one third opening (13, 14) that is an inlet for gas containing a component to be measured, **characterized in that**
the housing (1) includes two or more third openings (13, 14) that are inlets for gas containing a component to be measured,
at least one of the third openings (13, 14) includes a shutter that can be opened/closed, and
the shutter of one of the third openings (13) is configured to be closed when the gas flows in through another third opening (14).

2. The measurement apparatus (100) according to claim 1, wherein:
the substrate (3) includes a pump (5) configured to allow air that flows in through the third opening (13, 14) to be discharged from the first opening (11).

3. The measurement apparatus (100) according to claim 1 or 2, wherein:
the odor sensor (4) is configured of two or more Quartz Crystal Microbalance sensors.

## Patentansprüche

1. Messvorrichtung (100), mit
einem mit einem Geruchssensor (4) versehenen Substrat (3), und
einem Gehäuse (1), das das Substrat (3) aufnimmt und eine erste Öffnung (11), die ein Luftauslass ist, eine zweite Öffnung (12), die ein Lufteinlass ist, und mindestens eine dritte Öffnung (13, 14), die ein Einlass für Gas ist, das eine zu messende Komponente enthält, aufweist, **dadurch gekennzeichnet, dass**
das Gehäuse (1) zwei oder mehr dritte Öffnungen (13, 14) aufweist, die Einlässe für Gas sind, das eine zu messende Komponente enthält,
mindestens eine der dritten Öffnungen (13, 14) einen Verschluss aufweist, der geöffnet/geschlossen werden kann, und
der Verschluss einer der dritten Öffnungen (13) so konfiguriert ist, dass er geschlossen wird, wenn das Gas durch eine andere dritte Öffnung (14) einströmt.

2. Messvorrichtung (100) gemäß Anspruch 1, wobei:
das Substrat (3) eine Pumpe (5) aufweist, die konfiguriert ist, um das Auslassen von Luft, die durch die dritte Öffnung (13, 14) einströmt, aus der ersten Öffnung (11) zu ermöglichen.

3. Messvorrichtung (100) gemäß Anspruch 1 oder 2, wobei:
der Geruchssensor (4) konfiguriert ist aus zwei oder mehr Quarzkristall-Mikrowaagensensoren.

## Revendications

1. Appareil de mesure (100), comprenant :
un substrat (3) muni d'un capteur olfactif (4) ; et
un boîtier (1) accueillant le substrat (3) et incluant une première ouverture (11) qui est une sortie d'air, une deuxième ouverture (12) qui est une entrée d'air, et au moins une troisième ouverture (13, 14) qui est une entrée pour un gaz contenant un composant à mesurer, **caractérisé en ce que**
le boîtier (1) inclut deux ou plusieurs troisièmes ouvertures (13, 14) qui sont des entrées pour le gaz contenant un composant à mesurer,
au moins une des troisièmes ouvertures (13, 14) inclut un obturateur qui peut être ouvert/fermé, et
l'obturateur de l'une des troisièmes ouvertures (13) est configuré pour être fermé lorsque le gaz entre par une autre troisième ouverture (14).

2. Appareil de mesure (100) selon la revendication 1, dans lequel :
le substrat (3) inclut une pompe (5) configurée pour permettre à l'air qui entre par la troisième ouverture (13, 14) d'être évacué par la première ouverture (11).

3. Appareil de mesure (100) selon la revendication 1 ou 2, dans lequel :
le capteur olfactif (4) est configuré avec deux ou plusieurs capteurs à microbalance à cristal de quartz.
